Europäisches Patentamt

European Patent Office

Office européen des brevets

(11) Veröffentlichungsnummer: **0 402 591**
**A1**

(12) # EUROPÄISCHE PATENTANMELDUNG

(21) Anmeldenummer: **90107186.0**

(51) Int. Cl.⁵: **A61B 7/02**

(22) Anmeldetag: **14.04.90**

(30) Priorität: **16.06.89 DE 8907346 U**

(43) Veröffentlichungstag der Anmeldung:
**19.12.90 Patentblatt 90/51**

(84) Benannte Vertragsstaaten:
**CH DE FR LI**

(71) Anmelder: **KIRCHNER & WILHELM GMBH & CO.**
**Eberhardstrasse 56**
**D-7144 Asperg 1(DE)**

(72) Erfinder: **Kirchner, Regina**
**Graf-Hartmann-Strasse 30 a**
**D-7145 Markgröningen(DE)**
Erfinder: **Kirchner, Ulrich**
**Paulinenstrasse 25**
**D-7145 Markgrönigen(DE)**

(74) Vertreter: **Schmid, Berthold et al**
**Patentanwälte Dipl.-Ing. B. Schmid Dr. Ing. G.**
**Birn Falbenhennenstrasse 17**
**D-7000 Stuttgart 1(DE)**

(54) **Stethoskop.**

(57) Um bei einem Stethoskop, dessen Schlauchan-schlußkörper (4) wenigstens einen Schalltrichter (5) und/oder ein Membranteil (7) aufweist, den Herstel-lungsaufwand sowie die Herstellungskosten zu ver-ringern, ist jeder Schalltrichter (5) und/oder trichterförmige Grundkörper (6) des Membranteils (7) einstückig mit dem Schlauchanschlußkörper (4) gefer-tigt. Vorzugsweise kann bei diesem Stethoskop mit-tels eines in zwei Arbeitsstellungen einrastbaren schlauchanschlußstutzens (2) von dem Membranteil (7) auf den Schalltrichter (5) umgeschaltet werden.

EP 0 402 591 A1

## Stethoskop

Die Erfindung bezieht sich auf ein Stethoskop, dessen aus Kunststoff gefertigter Schlauchanschlußkörper wenigstens einen Schalltrichter oder ein Membranteil oder einen Schalltrichter und ein Membranteil aufweist und mit einem Schlauchanschlußstutzen versehen ist. Bei einem bekannten Stethoskop dieser Art werden der Schalltrichter und der trichterförmige Grundkörper auf den Schlauchanschlußkörper aufgesteckt und dann jeweils mittels eines hohlnietartigen Befestigungselements festgehalten.

Die Aufgabe der Erfindung besteht nun darin, ein Stethoskop der eingangs genannten Art so weiterzubilden, daß die Teilezahl reduziert und damit der Herstellungsaufwand sowie die Herstellungskosten verringert werden können.

Zur Lösung dieser Aufgabe wird erfindungsgemäß vorgeschlagen, daß das Stethoskop gemäß dem Oberbegriff des Anspruchs 1 entsprechend dem kennzeichnenden Teil dieses Anspruchs ausgebildet ist. Weil bei diesem Stethoskop der trichterförmiger Grundkörper und/oder der Schalltrichter einstückig mit dem Schlauchanschlußstück gefertigt sind, entfällt nicht nur das Zusammensetzen der genannten Teile, sondern auch der jeweils notwendige Befestigungsvorgang. Insofern wird durch dieses Stethoskop die gestellte Aufgabe gut gelöst.

Wenn man dieses Stethoskop in schalltechnischer Hinsicht verbessern will, so kann dies in Weiterbildung der Erfindung dadurch geschehen, daß der Schalltrichter und/oder der trichterförmige Grundkörper an der Innenfläche, also an seiner hohlgewölbten, gegen die Schallquelle zu richtenden Innenfläche, lackiert oder mit Metall beschichtet ist bzw. sind. Die Metallbeschichtung kann man beispielsweise durch Aufdampfen relativ preiswert aufbringen. Dadurch läßt sich auch die angestrebte Metalldicke sicher erzeugen.

Eine andere Variante der Erfindung ist dadurch gekennzeichnet, daß sich vor der hohlgewölbten Innenfläche des trichterförmigen Grundkörpers und/oder des Schalltrichters eine hohlgewölbte Metallplatte befindet und diese an einem zentrischen Durchbruch unmittelbar mit dem Kunststoffmaterial des Schlauchanschlußkörpers verbunden ist. Die hohlgewölbten Flächen der Metallplatte bzw. Metallplatten und des zugeordneten Trichters bzw. des trichterförmigen Grundkörpers können parallel oder etwa parallel zueinander verlaufen. Auf jeden Fall weist beim Gebrauch dieses Stethoskops jeweils eine hohlgewölbte Innenfläche gegen die Schallquelle. Die Befestigung am zentrischen Durchbruch, über welchen der Schall in den Schlauchanschlußkörper eintreten kann, läßt sich in bekannter Weise vornehmen. Am Außenrand kann man die hohlgewölbte Metallplatte ebenfalls in bekannter Weise fixieren, beispielsweise mittels eines ihren Außenrand und denjenigen des Schalltrichters bzw. des trichterförmigen Grundkörpers übergreifenden Befestigungsrings.

Eine besonders bevorzugte Ausführungsform der Erfindung sieht vor, daß der Schlauchanschlußkörper einen zentrischen, in den Schalltrichter und/oder den trichterförmigen Grundkörper hineinragenden, rohrförmigen Befestigungsansatz aufweist, der den zugeordneten zentrischen Durchbruch der Metallplatte durchsetzt und insbesondere thermisch oder mittels Ultraschall umgebördelt oder umgeschlagen ist. Der rohrförmige Befestigungsansatz bewirkt zugleich eine Zentrierung der Metallplatte gegenüber dem Schalltrichter oder dem Grundkörper. Weder der Durchbruch noch der rohrförmige Befestigungsansatz müssen aber notwendigerweise einen kreisförmigen Querschnitt haben, vielmehr sind auch andere Querschnittsformen, beispielsweise eine Mehrkantform, möglich. Die Bohrung des rohrförmigen Befestigungsansatzes wählt man aber zweckmäßigerweise kreisrund.

Eine weitere Ausgestaltung der Erfindung besteht darin, daß sich zwischen dem Schalltrichter und/oder dem trichterförmigen Grundkörper und der zugeordneten Metallplatte ein am inneren und äußeren Rand geschlossener Hohlraum befindet. Die Metallplatte liegt also bei dieser Ausführungsform nicht unmittelbar am dahinter befindlichen trichterförmigen Grundkörper bzw. Schalltrichter an, sondern hat davon einen gewissen Abstand. Weil sowohl ihr innerer Bereich als auch ihr Außenrand jeweils festgehalten sind, entsteht dadurch ein Luftpolster, welches das Schwingungsverhalten der metallenen Platte gegenüber einer Ausführungsform mit unmittelbarer Anlage verändert.

Eine weitere besonders bevorzugte Ausführungsform der Erfindung ergibt sich aus Anspruch 7. Doppelt verwendbare Stethoskope sind im Prinzip bereits bekannt, und dies gilt auch für den Umschaltmechanismus. Letzterer kann in bekannter Weise ausgebildet sein. Dies ist beim Ausführungsbeispiel der Fall und wird in der nachfolgenden Zeichnungsbeschreibung näher erläutert.

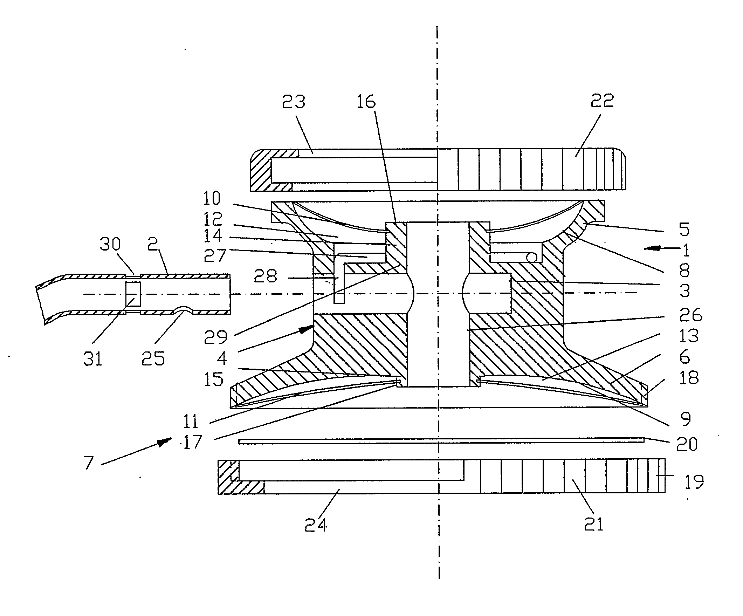

Die Zeichnung zeigt ein Ausführungsbeispiel der Erfindung in einer schematisierten, teilweise explosionsartigen Darstellung, teilweise im Halbschnitt und teilweise in einem vertikalen Längs mittelschnitt.

Das Stethoskop besteht in bekannter Weise aus einem Bruststück 1 und einem nicht dargestellten Ohrbügel bekannter Konstruktion sowie einem dazwischengeschalteten, sich am Ohrbügel gabelnden Verbindungsschlauch. Er ist mit dem Schlauchanschlußstutzen 2 verbunden. Letzterer ist beim Ausführungsbeispiel drehbar, aber unverschiebbar in einer Sackbohrung 3 eines Schlauchanschlußkörpers 4 gelagert. Der Schlauchanschlußkörper besteht in bekannter Weise aus Kunststoff. Erfindungsgemäß ist er einstückig mit einem Schalltrichter 5 und einem trichterförmigen Grundkörper 6 eines Membranteils 7 hergestellt. Falls es sich entgegen der Zeichnung nicht um ein doppelseitig verwendbares Stethoskop handelt, so ist der Schlauchanschlußkörper 4 entweder nur mit dem trichterförmigen Grundkörper 6 oder nur dem Schalltrichter 5 ausgestattet, und es kann dann auch auf die um die Längsachse drehbare Lagerung des Schlauchanschlußstutzens 2 im Bruststück 1 bzw. Schlauchanschlußkörper 4 verzichtet werden.

Die hohlgewölbte Innenfläche 8 des Schalltrichters 5 und/oder die hohlgewölbte Innenfläche 9 des trichterförmigen Grundkörpers 6 kann bzw. können mit einer Lackschicht oder Metallschicht überzogen sein. Die Metallschicht bringt man zweckmäßigerweise durch Aufdampfen an, während man die Lackschicht aufspritzt oder in anderer bekannter Weise anbringt.

Eine andere Ausgestaltung des Bruststücks 1 besteht darin, daß man vor der hohlgewölbten Innenfläche 8 bzw. 9 eine hohlgewölbte, zentrisch gelochte Metallplatte 10 am Schalltrichter 5 und/oder Metallplatte 11 am trichterförmigen Grundkörper 6 anbringt. Diese kann entweder wie beim Ausführungsbeispiel dargestellt im Abstand von der zugeordneten Innenfläche 8 bzw. 9 angebracht sein, oder aber in nicht gezeigter Weise unmittelbar daran anliegen, wobei dann die Außenfläche der Metallplatte 10 bzw. 11 entsprechend der Höhlung des zugeordneten Schlauchanschlußkörperteils geformt ist. Beim Ausführungsbeispiel entstehen jedenfalls Hohlräume 12 bzw. 13, welche das freie Schwingen der Metallplatte gegenüber dem Schalltrichter 5 bzw. trichterförmigen Grundkörper 6 gestatten.

Der Schalltrichter 5 besitzt einen zentrischen, rohrför sein Inneres hineinragenden Befestigungsansatz 14, der die zentrische Bohrung der Metallplatte 10 durchsetzt und zur Befestigung der letzteren dient. An der gegenüberliegenden Seite ist am trichterförmigen Grundkörper 6 ein Befestigungsansatz 15 gleicher Art vorgesehen. Die beiden Befestigungsansätze sind an ihren freien Enden in nicht gezeigter Weise absatzartig reduziert, wobei der Außendurchmesser des reduzierten Teils etwa dem Innendurchmesser der zentrischen Bohrung der zugeordneten Metallplatte 10 bzw. 11 entspricht. Nach dem Aufsetzen der Metallplatte 10 bzw. 11 wird das überstehende freie Ende jedes Befestigungsansatzes bleibend verformt, beispielsweise mit Hilfe von Ultraschall. Es entsteht dadurch ein umgebördelter Rand 16 bzw. 17, welcher die zugeordnete Metallscheibe innen sicher und vorzugsweise auch luftdicht festhält.

Der Außenrand jeder Metallplatte stützt sich vorzugsweise im Bereich des Trichterrandes am Schalltrichter 5 bzw. trichterförmigen Grundkörper 6 ab. Er wird mittels eines geeigneten Rings festgehalten und auch gegen die Trichterfläche bzw. 9 gedrückt. Wenn der trichterförmige Grundkörper 6 beispielsweise mit einem Bolzengewinde 18 versehen ist, so verwendet man einen Schraubring 19 mit entsprechendem Muttergewinde. Außerdem wird zwischen dem Schraubring 18 und die hohlgewölbte Metallpalte 11 bzw. den freien Rand des Trichters 6 noch eine Membrane 20 bekannter Art geschaltet. Sie kann flach oder leicht gewölbt sein. Den Ring 19 kann man in diesem Falle mit einer Rändelung 21 ausstat

An der gegenüberliegenden Seite des Bruststücks ist beim Ausführungsbeispiel zu Demonstrationszwecken anstelle eines Schraubrings ein aufsprengbarer Ring 22 vorgesehen. Er untergreift den freien Randbereich des Schalltrichters 5 und besitzt ein zentrisches Loch 23 für den Schalldurchtritt. Ein entsprechendes Loch 24 ist am Schraubring 19 vorhanden.

Das in der Zeichnung rechte Ende des Schlauchanschlußstutzens 2 wird in die Sackbohrung 3 etwa bis zum Anschlag eingeschoben. Es besitzt eine radiale Bohrung 25, die bei korrekter Montage mit der zentrischen Längsbohrung 26 des Schlauchanschlußkörpers 4 korrespondiert, wenn sich der Schlauchanschlußstutzen 2 in einer seiner beiden Gebrauchs-Drehstellungen befindet.

Letztere können mit Hilfe einer sinnvollen Einrichtung relativ leicht aufgefunden werden. Zu diesem Zwecke ist in einen sich an die hohlgewölbte Innenfläche 8 des Schalltrichters 5 anschließenden Absatz eine kreisringförmige Feder 27 eingelegt, deren beide rechtwinklig abgebogenen freien Enden 28 je eine von zwei parallelen Bohrungen des Bodens 29 des Schlauchanschlußkörpers 4 durchsetzen und in die Sackbohrung 3 hineinragen.

Der Schlauchanschlußstutzen 2 ist mit einer umlaufenden Außennut 30 ausgestattet. Bei vollständig eingeschobenem Schlauchanschlußstutzen ist diese den Federenden 28 zugeordnet. Außerde der Abstand dieser beiden Enden so gewählt, daß eines links und das andere rechts am Röhrchen vorbei nach unten ragt. Dadurch bilden diese beiden Enden 28 in Verbindung mit der Ringnut 30 eine Sicherung gegen axiale Verschiebung.

Desweiteren ist im Bereich der Ringnut 30 am Schlauchanschlußstutzen 2 noch ein Durchbruch 31 oder eine entsprechende Abflachung angebracht. Sie sind gegenüber der radialen Bohrung 25 so plaziert, daß diese mit dem oberen oder dem unteren Ende der zentrischen Längsbohrung 26 korrespondiert, wenn einer der beiden Federschenkelenden 28 an der Abflachung anliegt, bzw. in den fensterartigen Durchbruch 31 eingreift. Somit sind die Umschaltstel lungen durch entsprechendes Einrasten relativ leicht auffindbar.

Die Feder 27 ist in geeigneter, nicht dargestellter Weise, gegen eine axiale Verschiebung parallel zum Befestigungsansatz 14 gesichert, indem man beispielsweise zwischen die Feder und den Innenrand der Metallplatte 10 ein Distanzstück entsprechender Form und Größe einlegt.

Aus dem Vorstehenden wird klar, daß man zunächst den Schlauchanschlußstutzen 2 in die Sackbohrung 3 einschiebt, anschließend die Feder 27 montiert und erst daraufhin die hohlgewölbte Metallplatte 10 montiert. Abschließend wird dann die Umbördelung 16 od. dgl. erzeugt. Die übrigen Teile können im Sinne der explosionsartigen Darstellung nacheinander montiert werden.

# Ansprüche

1. Stethoskop, dessen aus Kunststoff gefertigter Schlauchanschlußkörper wenigstens einen Schalltrichter oder ein Membranteil oder einen Schalltrichter und ein Membranteil aufweist und mit einem Schlauchanschlußstutzen versehen ist, dadurch gekennzeichnet, daß jeder Schalltrichter (5) und/oder trichterförmige Grundkörper (6) des Membranteils (7) einstückig mit dem Schlauchanschlußkörper (4) gefertigt ist, bzw. sind.

2. Stethoskop nach Anspruch 1, dadurch gekennzeichnet daß der Schalltrichter (5) und/oder der trichterförmige Grundkörper (6) an der Innenfläche (8) bzw. (9) lackiert oder mit Metall beschichtet ist bzw. sind.

3. Stethoskop nach Anspruch 1, dadurch gekennzeichnet, daß sich vor der hohlgewölbten Innenfläche (8) bzw (9) des trichterförmigen Grundkörpers (6) und/oder des Schalltrichters (5) eine hohlgewölbte Metallplatte (11, 10) befindet und diese an einem zentrischen Durchbruch unmittelbar mit dem Kunststoffmaterial (14, 15) des Schlauchanschlußkörpers (4) verbunden ist.

4. Stethopskop nach Anspruch 3, dadurch gekennzeichnet, daß der Schlauchanschlußkörper (4) einen zentrischen, in den Schall trichter (5) und/oder den trichterförmigen Grundkörper (6) hineinragenden rohrförmigen Befestigungsansatz (14 bzw. 15) aufweist, der den zugeordneten zentrischen Durchbruch der Metallplatte (10 bzw. (11) durchsetzt und insbesondere thermisch oder mittels Ultraschall umgebördelt (16, 17) ist.

5. Stethoskop nach Anspruch 3 oder 4, dadurch gekennzeichnet, daß sich zwischen dem Schalltrichter (5) und/oder dem trichterförmigen Grundkörper (6) und der zugeordneten Metallplatte (10 bzw. 11) ein am inneren und äußeren Rand geschlossener Hohlraum (12 bzw. 13) befindet.

6. Stethoskop nach wenigstens einem der vorhergehe en Ansprüche, gekennzeichnet durch eine Ausbildung als umschaltbares Stethoskop mit einem Membranteil (7) und einem vorzugsweise kleineren Schalltrichter (5) sowie einem um seine Längsachse drehbaren, insbesondere in den beiden Arbeitsstellungen einrastbaren Schlauchanschlußstutzen (2).

EP 0 402 591 A1

Europäisches
Patentamt

**EUROPÄISCHER RECHERCHENBERICHT**

Nummer der Anmeldung

EP 90 10 7186

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | AU-B- 504 509 (METPRO PTY. LTD.) <br> * Seite 3, Zeile 21 - Seite 5, Zeile 3; Figuren 1-5 * | 1,6 | A 61 B 7/02 |
| A | | 3,5 | |
| | --- | | |
| X | US-A-3 224 526 (R.E. WEBER) <br> * Spalte 2, Zeile 24 - Spalte 3, Zeile 55; Figuren 1,2 * | 1,6 | |
| | --- | | |
| X | WO-A-8 100 353 (MINNESOTA MINING AND MANUFACTURING CO.) <br> * Seite 6, Zeile 18 - Seite 9, Zeile 5; Figuren 1-5 * | 1,6 | |
| | --- | | |
| X | EP-A-0 119 870 (MINNESOTA MINING AND MANUFACTURING CO.) <br> * Seite 6, Zeile 20 - Seite 9, Zeile 38; Figuren 1-3 * | 1,6 | |
| | ----- | | |

**RECHERCHIERTE SACHGEBIETE (Int. Cl.5)**

A 61 B 7/00
G 10 K 11/00

Der vorliegende Recherchenbericht wurde für alle Patentansprüche erstellt

| Recherchenort | Abschlußdatum der Recherche | Prüfer |
|---|---|---|
| BERLIN | 30-08-1990 | WEIHS J.A. |

EPO FORM 1503 03.82 (P0403)

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument